# EUROPEAN PATENT APPLICATION

(11) **EP 2 617 374 A1**
(43) Date of publication of application: **24.07.2013**
(21) Application number: 13151772.4
(22) Date of filing: 18.01.2013
(51) Int. Cl.: A61B 17/34

(54) **Wound protector including flexible and rigid liners**

(30) Priority: 19.01.2012 US 201261588324 P; 28.12.2012 US 201213729085
(71) Applicant: Covidien LP, Mansfield, MA 02048 (US)
(72) Inventor: Smith, Robert C., Middlefield, Connecticut 06455 (US)
(74) Representative: Soames, Candida Jane

(57) **Abstract**

A wound protection system is disclosed. The wound protection system includes a wound protector and a seal anchor member that are engageable with one another and insertable into a bodily opening. The wound protection system protects the bodily opening from contamination while facilitating access to underlying bodily structures within the surgical site.

## Description

### CROSS REFERENCE TO RELATED APPLICATION

The present application claims the benefit of and priority to U.S. Provisional Application Serial No. 61/588,324, filed on January 19, 2012, the entire contents of which are incorporated herein by reference.

### BACKGROUND

### 1. Technical Field

The present disclosure relates to a surgical device for protecting a bodily opening, e.g., a wound or naturally occurring orifice, through which a surgical procedure is performed. More particularly, a wound protector for use during a minimally invasive surgical procedure is disclosed.

### 2. Background of Related Art

Minimally invasive surgery, e.g., laparoscopic, endoscopic, and thoroscopic surgery, has many advantages over traditional open surgeries. In particular, minimally invasive surgery eliminates the need for a large incision, thereby reducing discomfort, recovery time, and many of the deleterious side effects associated with traditional open surgery.

Minimally invasive surgeries are performed through small openings in a patient's skin. These openings may be incisions in the skin or may be naturally occurring body orifices (e.g., mouth, anus, or vagina).

When an incision is required, the initial puncture is usually very small so that a needle or trocar can achieve the desired penetration without excessive damage to nearby tissue. It may be necessary for the initial access hole to be subsequently enlarged to provide a working diameter to permit introduction of surgical instruments and the performance of the desired medical procedure.

As with any incision, the creation of a wound presents a risk of contamination of the wound, e.g., a bacterial infection. It may therefore be desirable to protect the wound edges with a wound protector to minimize contamination of the wound. In addition, during a surgical procedure, it may be desirable to protect the inner surfaces of a bodily opening to minimize damage and/or minimize discomfort. A continuing need exists for improved bodily opening protection devices.

### SUMMARY

Disclosed herein is a wound protection system. In a first embodiment, the wound protection system includes a liner that is insertable into a bodily opening and includes a longitudinally extending lumen. A wound protector is insertable into the lumen of the liner and a seal anchor is insertable into the wound protector. In another embodiment, a wound protection system includes a seal anchor member including a port member, a housing, member, and a cover. The port member is placeable within the housing member, and the cover is secureable to the housing member. The port member includes one or more longitudinally extending ports. The cover includes an aperture to facilitate access to the one or more longitudinally extending ports of the port member through the cover. The wound protection system also includes a wound protector that is insertable into a tissue tract. The wound protector includes a longitudinally extending lumen into which the seal anchor member is placeable.

In an embodiment, the wound protection system includes a liner that is placeable within a tissue tract and that is transitionable between a contracted condition and an expanded condition. In the contracted condition, placement of the liner is facilitated by its reduced width. In the expanded condition, access to the underlying body cavity is facilitated by the increased width of the lumen of the liner. A tool or expander is provided to facilitate transitioning of the liner to the expanded condition. The tool or expander may include a balloon that expands after placement within the lumen of the liner.

A method for deploying a wound protection device into a bodily opening includes providing a wound protection system such as the one described above. The liner is placed within the bodily opening and the wound protector is translated distally through the lumen of the liner. The seal anchor member is then inserted into the wound protector, and one or more surgical instruments are inserted into the one or more longitudinally extending ports of the seal anchor member. The desired surgical procedure is performed, and then the surgical instruments are removed, and the wound protection system is removed from the bodily opening.

These and other embodiments of the present disclosure will be described in detail below with reference to the accompanying figures.

### BRIEF DESCRIPTION OF THE DRAWINGS

Embodiments of the present disclosure are described herein with reference to the accompanying drawings, wherein:

Fig. 1 is a perspective view of an embodiment wound protector system in accordance with the present disclosure with parts separated;

Fig. 2 is a perspective view of an embodiment of a wound protector system in accordance with the present disclosure with parts separated;

Fig. 3 is a perspective view of an embodiment of a wound protector system in accordance with the present disclosure with parts separated;

Fig. 4 is a perspective view of an embodiment of a wound protector system in accordance with the present disclosure with parts separated;

Fig. 4A is a front view of the wound protector system of Fig. 4;

Fig. 4B is a top view of a flexible liner shown in a first condition;

Fig. 4C is a top view of the flexible liner of FIG. 4B shown in a second condition;

Fig. 4D is a top view of an embodiment of a flexible liner shown in a first condition;

Fig. 4E is a top view of the flexible liner of Fig. 4D shown in a second condition;

Fig. 5 is a cross-sectional view of an embodiment of a wound protector system in accordance with the present disclosure;

Fig. 6 is a cross-sectional view of a wound protector shown positioned within an opening within tissue;

Fig. 7 is a cross-sectional view of the wound protector of Fig. 5 shown with a liner and tool partially positioned therein; and

Fig. 8 is a cross-sectional view of the wound protector of Fig. 5 shown with the liner of Fig. 5 and a seal anchor partially inserted therein.

### DETAILED DESCRIPTION

Embodiments of the present disclosure will now be described in detail with reference to the drawings, wherein the reference numerals identify similar or identical elements. In the drawings and in the description that follows, the term "proximal," will refer to the end of a device that is closest to the operator, while the term "distal" will refer to end of the device that is farthest from the operator.

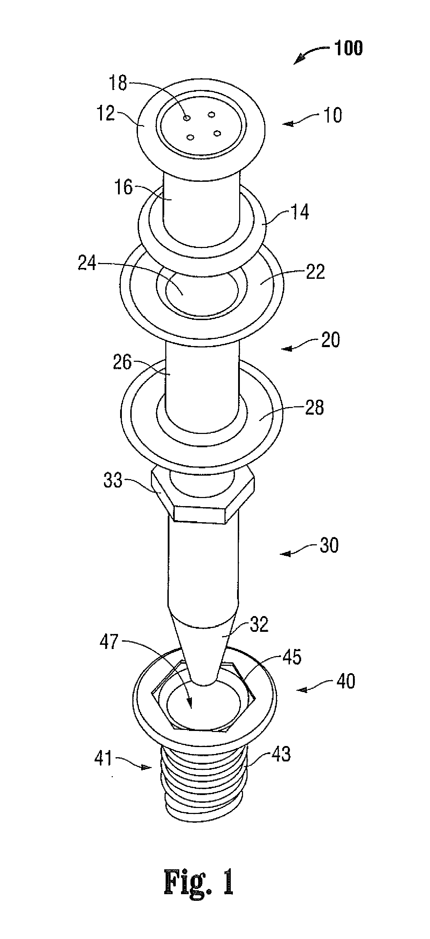

A first embodiment of a wound protector system will be described with reference to Fig. 1. As shown in Fig. 1, a wound protector system 100 includes a liner 40, a wound protector 20, and a seal anchor member 10 that are operatively engageable with one another for placement within a bodily opening. The wound protector system 100 also includes a tool 30 that is configured and adapted to engage the liner 40.

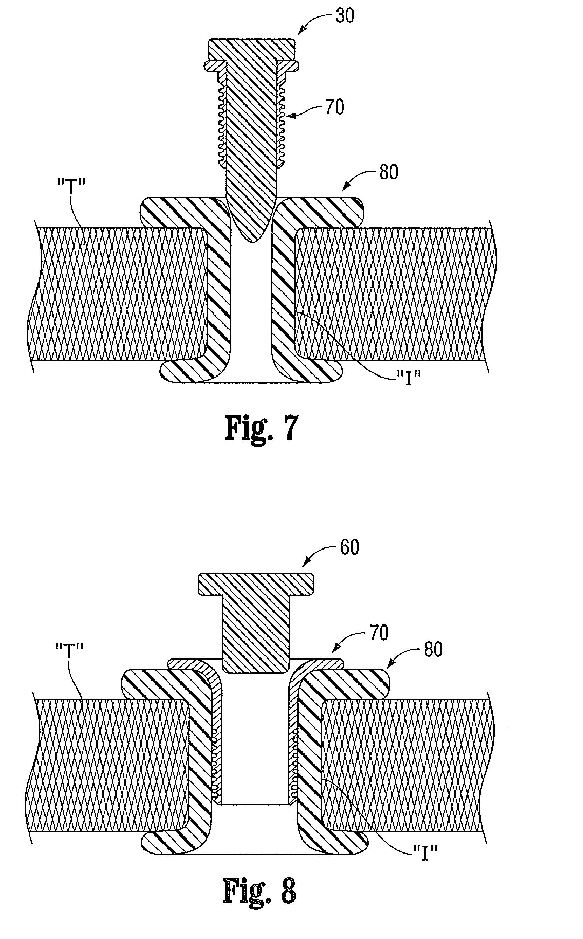

The liner 40 is configured and adapted to be placed within a tissue tract "I" that is defined within a tissue "T" (Fig. 8). The liner 40 functions to protect the sides and edges of the tissue tract "I" that is defined within the tissue "T". The liner 40 may form a seal with the tissue tract "I" to minimize contamination of the sides or edges of the tissue tract "I". The liner 40 may be formed from a material having non-reactive or inert properties, e.g., polyurethane. Moreover, the liner 40 may be formed from a material having anti-bacterial or anti-microbial properties, such as a material having an oligodynamic effect, e.g., a material including silver. The liner 40 may also be infused or lined with an anti-bacterial or anti-microbial substance, e.g., gel. The liner 40 includes an elongated section 41 that includes threading 43 disposed along the length of the elongated section 41.

The tool 30 is configured and adapted to engage the liner 40 to facilitate placement of the liner 40 within the tissue tract "I" within the tissue "T". The tool 30 includes a tapered distal end 32 that facilitates insertion of the tool 30 into a lumen 47 defined within the liner 40. The tool 30 also includes a proximal end 33 that is configured to engage a complimentary proximal end 45 of the liner 40. For example, as shown in Fig. 1, the proximal end 33 of the tool 30 may have a regular hexagon shape that fits within a regular hexagon shaped groove within the proximal end 45 of the liner 40. The proximal end 33 of the tool 30 may have a length that is greater than the depth of the proximal end 45 of the liner 40, thereby permitting grasping of the proximal end 33 of the tool 30 while the tool 30 is seated within the liner 40. The tool 30 may be used to push the liner 40 or to rotate the liner to cause the threading 43 to engage the surfaces of the tissue tract "I" and cause placement of the liner 40 therein. By operatively engaging the proximal end 32 of the tool 30 with the proximal end 45 of the liner 40, rotation of the tool 30 will cause a corresponding rotation of the liner 40, thereby engaging the threading 43 with the tissue tract "I" when the liner 40 is placed within the tissue tract "I". As the threading 43 is engaged with the tissue tract "I", the interaction of the threading 43 with the tissue tract "I" facilitates securing the liner 40 within the tissue tract "I".

After the liner 40 is placed within the tissue tract "I" of the tissue "T", the wound protector 20 is inserted into the lumen 47 of the liner 40. The wound protector 20 includes a proximal end 22, a distal end 28, and an intermediate section 26 disposed between the proximal and distal ends 22, 28. A lumen 24 extends longitudinally through the length of the wound protector. The wound protector 20 may be formed from a compressible material, such that the wound protector compresses as it is inserted through the liner 40. Once fully placed within the liner 40, the intermediate section 26 of the wound protector is positioned within the elongated section 41 of the liner 40, and the elongated section 41 is positioned between the proximal and distal ends 22, 28 of the wound protector 20. In an at rest state, the diameters of the proximal and distal ends 22, 28 of the wound protector 20 may be larger than the diameter of the elongated section 41 of the liner 40. The larger diameter of the distal end 28 of the wound protector may facilitate securing of the wound protector 20 relative to the liner 40.

The seal anchor member 10 is insertable into the wound protector 20. The seal anchor member 10 is formed from a compressible material and includes a proximal portion 12, a distal portion 14, and an intermediate portion 16 positioned therebetween. One or more longitudinally extending ports 18 extend through and between the proximal and distal portions 12, 14 of the seal anchor member 10. Each port 18 is configured and adapted to facilitate reception of a surgical instrument therethrough. An example of a seal anchor member is found in U.S. App. No. 12/244,024, filed on October 2, 2008, the entire contents of which is hereby incorporated by reference.

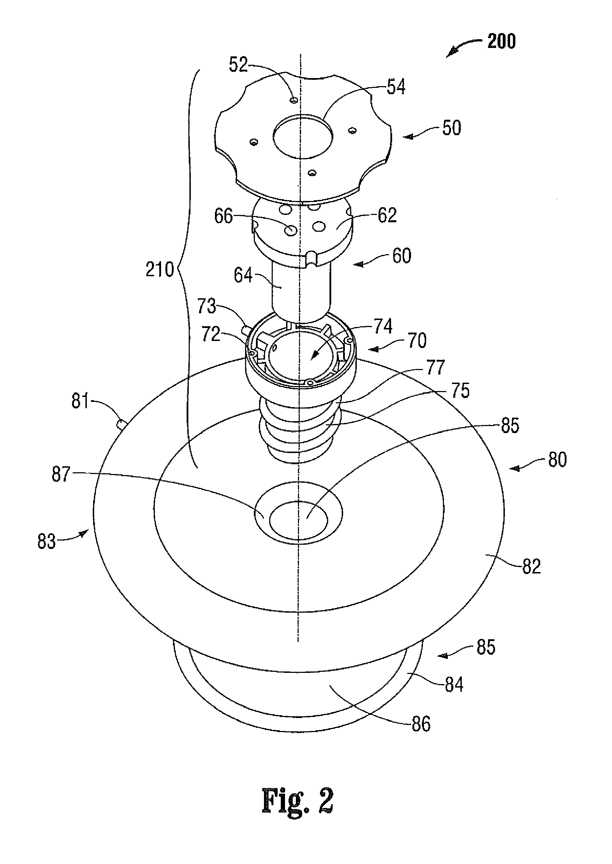

A second embodiment of a wound protector system will now be described with reference to Fig. 2. As shown in Fig. 2, a wound protector system 200 includes a seal anchor member 210 and a wound protector 80. The seal anchor member 210 includes a port member 60, a housing member 70, and a cover 50 The port member 60 includes a proximal end 62, an elongated intermediate section 64, and one or more longitudinally extending ports 66. The elongated intermediate section 64 of the port member 60 is configured and adapted to be received within the lumen 74 of housing member 70. The housing member 70 includes an insufflation port 73 to facilitate insufflation of the surgical working site. The housing member 70 includes a tubular section 75. The tubular section 75 may include threading 77. A cover 50 is securable to the housing member 70. The housing member 70 includes securement holes 72 that correspond with securement holes 52 of the cover 50. The securement holes 52, 72 are configured and adapted to receive a screw or other suitable fastener (not shown) to facilitate operably coupling the cover 50 to the housing member 70. The cover includes an aperture 54 to facilitate access to ports 66 through the cover 60.

The seal anchor member 210 is insertable into the wound protector 80. The wound protector 80 includes a longitudinally extending port 85. The longitudinally extending port 85 is configured and adapted to receive the seal anchor member 210 therein. The proximal end of the longitudinally extending port 85 may include a tapered region 87 to facilitate insertion of the seal anchor member 210 within the longitudinally extending port 85. The threading 77 of the tubular section 75 of the housing member 70 may facilitate anchoring of the seal anchor member 210 within the wound protector 80. The wound protector 80 includes a proximal section 83 that includes a balloon 82 and a distal section 85 that includes a guide member 84 that forms a flange to facilitate anchoring of the wound protector within the tissue tract "I" of the tissue "T". Inflation of the balloon 82 may be accomplished by operably coupling a source of insulation fluid (e.g., gas or liquid) to a port 81 that is fluidly coupled to the balloon 82.

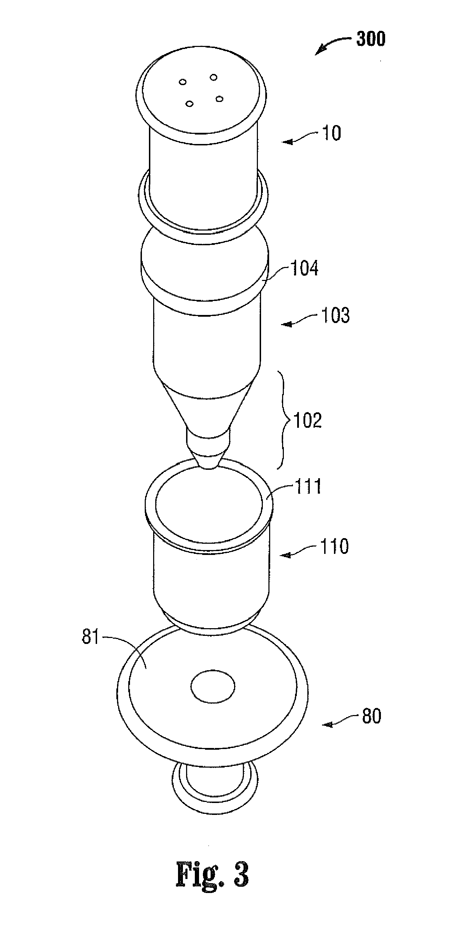

A third embodiment of a wound protector system will now be described with reference to Fig. 3. As shown in Fig. 3, a wound protector system 300 includes a wound protector 80 that is positionable within tissue tract "I" of tissue "T". The wound protector system 300 includes the seal anchor member 10 that is configured and adapted to be placed within a liner 110, and the wound protector 80. A tapered tool 103 includes a tapered region 102 to facilitate placement of the liner 110 into the wound protector 80. The tool 103 includes a proximal end 104 that engages the proximal lip 111 of the liner 110. Once the liner 110 is inserted into the wound protector 80, the seal anchor member 10 is placed within the liner 110.

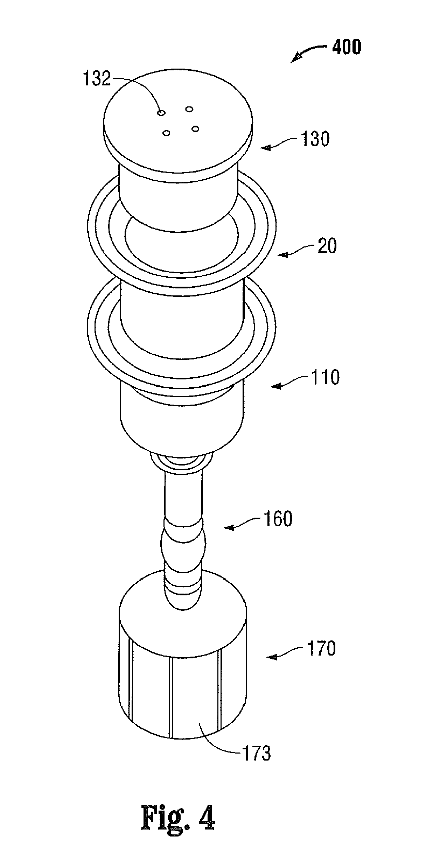

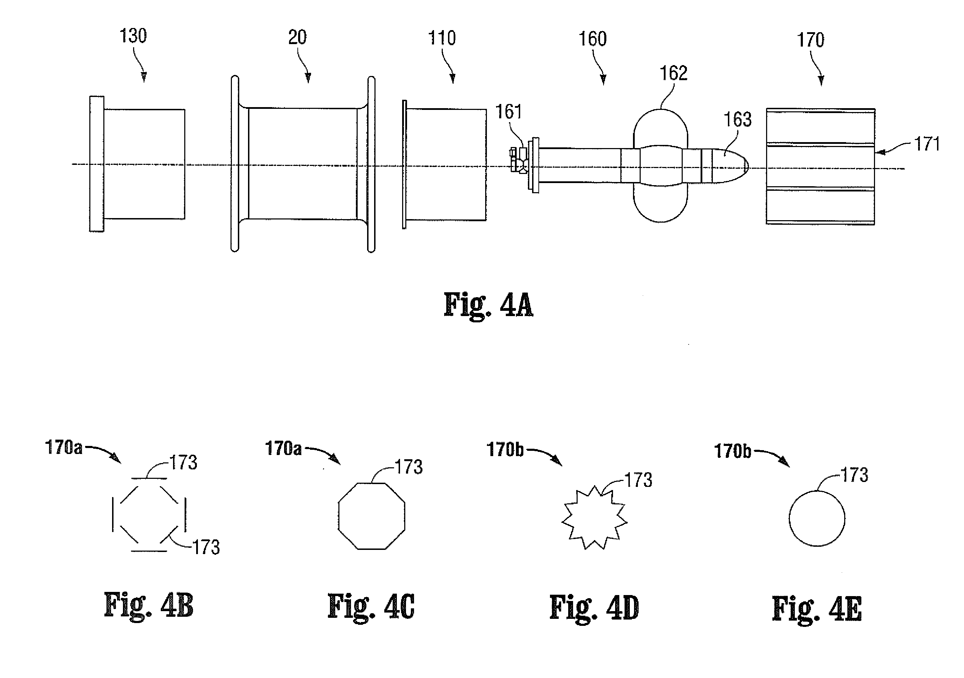

A fourth embodiment of a wound protector system will now be described with reference to Figs. 4 and 4A. A wound protector system 400 includes the seal anchor member 20 that is placeable within liner 110, which is placed in turn in flexible liner 170. The flexible liner 170 includes a longitudinally extending lumen 171. Expansion of the flexible liner 170 is achievable through several means. For example, the flexible liner 170 can be expanded by stretching and deforming the flexible liner 170 such that it transitions to an expanded state. In embodiments, expansion of the flexible liner 170 results in a permanent deformation and stretching of the flexible liner 170 to an expanded state.

In an embodiment of a flexible liner, a flexible liner 170A may include a plurality of interconnected members 173 that are repositionable, e.g., slidable, relative to one another such that the interconnected members 173 can be moved apart to expand the flexible liner 170. For example, the interconnected members 173 may be slidably movable relative to one another such that the flexible liner 170 is transitionable from a first state as shown in Fig. 4B to a second state as shown in Fig. 4C. In another embodiment, flexible liner 170B includes a plurality of interconnected members 173 that are foldable relative to one another to collapse (Fig. 4D) and expand (Fig. 4E) in an accordion-like fashion.

Subsequent to placement of the flexible liner 170 within the tissue tract "I" of the tissue "T" in a generally collapsed or contracted state, an expander 160 may be placed within lumen 171 to expand the flexible liner 170. The expander 160 includes a balloon 162 and a valve 161 that is fluidly coupled to the balloon 162 to facilitate the flow of fluid, e.g., gas or liquid, into the balloon 162. A tapered distal region 163 of the expander 160 may facilitate insertion of the expander 160 into the lumen 171 of the flexible liner 170.

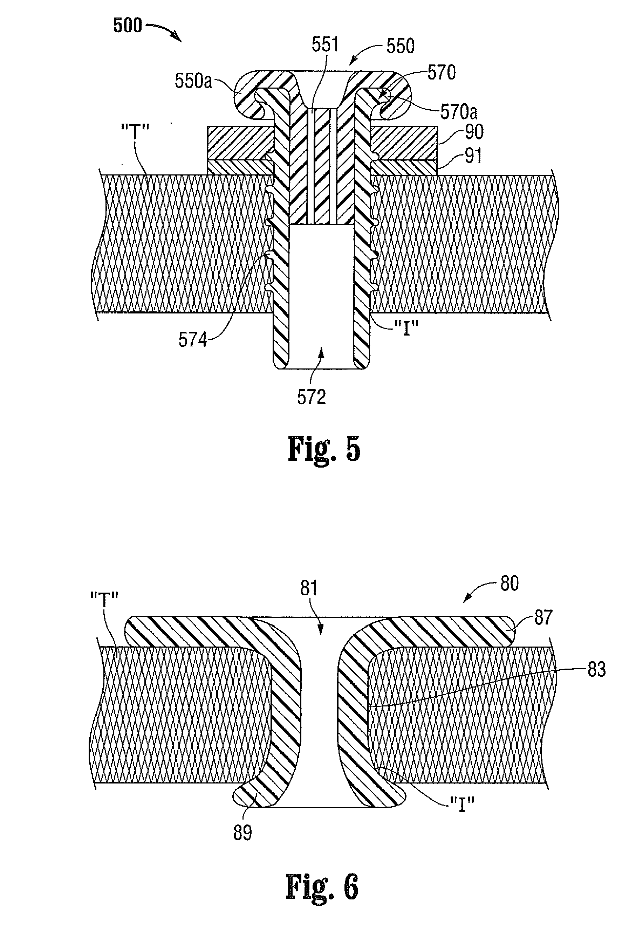

A fifth embodiment of a wound protector system will now be described with reference to Fig. 5. As shown in Fig. 5, a wound protector system 500 includes a wound protector 120 that is positionable within tissue tract "I" of tissue "T".

The wound protector system 500 includes a sheath 570 defining a longitudinally extending lumen 572. The sheath 570 includes threading 574 to facilitate frictional engagement and securement of the sheath 570 within the tissue tract "I" of tissue "T". A gasket 91 may be placed about the proximal portion of the sheath 570 to inhibit loss of insufflation gas between the sheath 570 and the tissue tract "I" of tissue "T". A nut 90 may be placed atop of the gasket 91 to facilitate maintenance of the sheath 570 and the gasket 91 in the desired positions relative the tissue "T". A port member 550 is insertable into lumen 572. The port member includes one or more longitudinally extending ports 551, each adapted and configured for the substantially sealed reception of a surgical instrument. The port member 550 includes a proximal portion 550a that is configured and adapted to be secured onto the proximal portion 570a of the sheath 570. For example, the proximal portion 550a of the port member 550 can snap onto the proximal portion 570a of the sheath 570.

A method of placing a wound protection system into a tissue tract "T" will now be described with reference to Figs. 6-8. As shown in Fig. 6, wound protector 80 is placed within the tissue tract "I", e.g., an incision or a naturally occurring orifice, in the tissue "T". A substantially rigid cannula, such as housing member 70, may then be placed within the wound protector 80 to facilitate maintaining the wound protector 80 in an expanded state. Insertion of the housing member 70 may be facilitated by using tool 30 as shown in Fig. 7.

As shown in Fig. 8, once the housing member 70 is placed within the wound protector 80, port member 60 may be placed within the housing member 70. As needed, the port member 60 may be removed and re-inserted during the course of the surgical procedure. Once the procedure is completed, the tool 30 may be used to facilitate removal of the housing member 70 by placing the tool 30 within the housing member 70, frictionally engaging the housing member 70, and withdrawing the tool 30 along with the housing member 70 from the wound protector 80.

Each of the embodiments described above are provided for illustrative purposes only. It will be understood that various modifications may be made to the embodiments of the present disclosure. Therefore, the above description should not be construed as limiting, but merely as exemplifications of embodiments. Those skilled in the art will envision other modifications within the scope and spirit of the present disclosure.

The invention may be described by reference to the following numbered paragraphs:-
1. A wound protection system, comprising:
   a liner, the liner insertable into a bodily opening, the liner including a longitudinally extending lumen;
   a wound protector insertable into the lumen of the liner; and
   a seal anchor member insertable into the wound protector.
2. A wound protection system, comprising:
   a seal anchor member including a port member, a housing member, and a cover, the port member placeable within the housing member, and the cover secureable to the housing member, the port member including one or more longitudinally extending ports, the cover including an aperture to facilitate access to the one or more longitudinally extending ports through the cover; and
   a wound protector including a longitudinally extending lumen, the seal anchor member placeable within the wound protector.
3. The wound protection system of paragraph 1, wherein the liner is transitionable between a contracted condition and an expanded condition, the liner defining a first width in the contracted condition and a second width in the expanded condition, the second width greater than the first width.
4. The wound protection device of paragraph 1, further comprising a tool configured and adapted to engage the liner to facilitate transitioning of the liner to the expanded condition.
5. The wound protection device of paragraph of paragraph 3, wherein the tool includes a balloon transitionable between a first width and a second width.
6. The wound protection device of paragraph 1, further comprising a tool configured and adapted to facilitate placement of the liner within a tissue tract.
7. The wound protection device of paragraph 1, wherein the tool includes a proximal portion and the liner includes a proximal portion, the proximal portions of the liner and the tool having a corresponding configuration.
8. A method for deploying a wound protection device into a bodily opening, comprising:
   providing a wound protection system, the wound protection system including:
      a liner, the liner insertable into a bodily opening, the liner including a longitudinally extending lumen;
      a wound protector insertable into the lumen of the liner; and
      a seal anchor member insertable into the wound protector;
   placing the liner within the bodily opening;
   distally translating the wound protector through the lumen of the liner;
   inserting the seal anchor member into the wound protector, the seal anchor member including one or more longitudinally extending ports;
   placing surgical instruments through the one or more longitudinally extending ports;
   performing a desired surgical procedure;
   removing the surgical instruments; and
   removing the wound protection system from the bodily opening.

## Claims

1. A wound protection system, comprising:
a liner, the liner insertable into a bodily opening, the liner including a longitudinally extending lumen;
a wound protector insertable into the lumen of the liner; and
a seal anchor member insertable into the wound protector.

2. A wound protection system, comprising:
a seal anchor member including a port member, a housing member, and a cover, the port member placeable within the housing member, and the cover secureable to the housing member, the port member including one or more longitudinally extending ports, the cover including an aperture to facilitate access to the one or more longitudinally extending ports through the cover; and
a wound protector including a longitudinally extending lumen, the seal anchor member placeable within the wound protector.

3. The wound protection system of claim 1, wherein the liner is transitionable between a contracted condition and an expanded condition, the liner defining a first width in the contracted condition and a second width in the expanded condition, the second width greater than the first width.

4. The wound protection device of claim 3, further comprising a tool configured and adapted to engage the liner to facilitate transitioning of the liner to the expanded condition.

5. The wound protection device of claim of claim 4, wherein the tool includes a balloon transitionable between a first width and a second width.

6. The wound protection device of any preceding claim further comprising a tool configured and adapted to facilitate placement of the liner within a tissue tract.

7. The wound protection device of claim 6, wherein the tool includes a proximal portion and the liner includes a proximal portion, the proximal portions of the liner and the tool having a corresponding configuration.

8. A method for deploying a wound protection device into a bodily opening, comprising:
providing a wound protection system, the wound protection system including:
a liner, the liner insertable into a bodily opening, the liner including a longitudinally extending lumen;
a wound protector insertable into the lumen of the liner; and
a seal anchor member insertable into the wound protector;
placing the liner within the bodily opening;
distally translating the wound protector through the lumen of the liner;
inserting the seal anchor member into the wound protector, the seal anchor member including one or more longitudinally extending ports;
placing surgical instruments through the one or more longitudinally extending ports;
performing a desired surgical procedure;
removing the surgical instruments; and
removing the wound protection system from the bodily opening.
